# FASCICULE DE BREVET EUROPEEN

(11) **EP 4 134 659 B1**
(45) Date de publication et mention de la délivrance du brevet: **30.04.2025**
(21) Numéro de dépôt: 21191299.3
(22) Date de dépôt: 13.08.2021
(51) Int. Cl.: G04D 7/04, G04D 7/00, G04D 5/00, G01N 21/64

(54) **ENSEMBLE MODULAIRE PORTATIF POUR INSPECTER DANS UNE PIÈCE D HORLOGERIE LA PRÉSENCE D'UN AGENT DE LUBRIFICATION OU D'UN ÉPILAME**
TRAGBARE MODULARE EINHEIT ZUR UNTERSUCHUNG EINER UHR AUF DAS VORHANDENSEIN EINES SCHMIERSTOFFS ODER EPILAMS
PORTABLE MODULAR ASSEMBLY FOR INSPECTING IN A TIMEPIECE THE PRESENCE OF A LUBRICATION AGENT OR AN EPILAME COATING

(43) Date de publication de la demande: 15.02.2023
(73) Titulaire: The Swatch Group Research and Development Ltd, 2074 Marin (CH)
(72) Inventeur: DINH, Jean-Bosco Thanh Khai, 1700 Fribourg (CH)
(74) Mandataire: ICB SA

(56) Documents cités:
- WO-A1-2006/077304
- WO-A2-2011/002209
- US-B2- 9 464 329
- ANONYMOUS: "Amazon.com: Qooltek Bracket/Headband Magnifying Glass Jewelry Clock Repair Loupe", 21 December 2017 (2017-12-21), XP055830882, Retrieved from the Internet <URL:https://www.amazon.com/Qooltek-Headband-Magnifying-Interchangeable-Upgraded/dp/B078JPRH2Y/ref=pd_day0_1/138-1801601-1391437?pd_rd_w=DA4Ox&pf_rd_p=8ca997d7-1ea0-4c8f-9e14-a6d756b83e30&pf_rd_r=QYXAGPXBYYXAZV8TPVYK&pd_rd_r=d432d58d-caf0-4b2f-ba63-8d138f2f620b&pd_rd_wg=4hIDj&pd_rd_i=B078JPRH2Y&th=1> [retrieved on 20210809]

## Description

### Domaine technique de l'invention

Le domaine de l'invention concerne l'inspection, la révision et l'entretien des pièces d'horlogerie.

Plus particulièrement, l'invention concerne un ensemble modulaire portatif permettant de combiner et de moduler différents équipements d'horlogerie d'usage courant pour inspecter une pièce d'horlogerie, et plus particulièrement pour contrôler la présence d'un agent de lubrification ou d'un épilame présentant des marqueurs fluorescents dans une pièce d'horlogerie sous excitation d'un flux lumineux de lumière blanche.

### Arrière-plan technologique

Il existe des agents de lubrification, tels que des huiles, des graisses, ou encore des épilames intégrant dans leur composition des marqueurs fluorescents facilitant leur visibilité lorsque ces agents de lubrification ou ces épilames sont soumis à une excitation lumineuse particulière.

L'utilisation de ces agents de lubrification, ou épilames, comportant de tels marqueurs fluorescents, est particulièrement appréciée pour l'inspection et l'entretien d'un mouvement d'horlogerie présentant un grand nombre de pièces et de points de lubrification.

Ces marqueurs fluorescents émettent une lumière verte sous l'excitation d'un flux lumineux présentant une plage de longueur d'onde comprise environ entre 350 nm et 480 nm.

Ainsi, il est possible d'exciter ces marqueurs fluorescents au moyen d'une source de lumière de rayonnement ultraviolet (UV) (longueur d'onde inférieure à 400nm) qui est notoirement nocive pour les yeux, ou encore au moyen d'une source de lumière bleue (longueur d'onde de l'ordre de 470 nm) en utilisant plusieurs filtres optiques pour obtenir la longueur d'onde, ou la plage de longueurs d'onde, désirée pour l'excitation des marqueurs fluorescents. Le document WO 2006/077304 A1 montre un appareil de détection portable permettant de détecter sur le terrain des éléments marqués par fluorescence.

Toutefois aucune de ces solutions n'est entièrement satisfaisante, car cela nécessite soit de se prémunir d'une protection visuelle particulière, soit de se prémunir d'une source de lumière bleue particulière pour exciter les marqueurs fluorescents.

Par conséquent, il existe un besoin pour faciliter l'inspection dans les pièces d'horlogerie de ces agents de lubrification ou de ces épilames présentant des marqueurs fluorescents.

### Résumé de l'invention

Dans ce contexte, l'invention propose un ensemble modulaire portatif pour inspecter dans une pièce horlogerie la présence d'un agent de lubrification ou d'un épilame présentant des marqueurs fluorescents, de conception simple, facile à mettre en œuvre et permettant de simplifier cette opération d'inspection intervenant lors des différentes maintenances et révisions des pièces d'horlogerie.

A cette effet, l'invention a pour objet un ensemble modulaire portatif pour inspecter dans une pièce d'horlogerie la présence d'un agent de lubrification, ou d'un épilame, présentant des marqueurs fluorescents, à partir d'un flux lumineux d'excitation de lumière blanche, ledit ensemble modulaire portatif étant caractérisé en ce qu'il comporte un boîtier optique, formant un premier module, une source de lumière blanche portative émettant un flux lumineux d'excitation, formant un deuxième module, un dispositif grossissant portatif, formant un troisième module ; ledit boîtier optique portant :
- un filtre d'excitation configuré pour sélectionner dans le flux lumineux d'excitation de ladite source de lumière blanche portative, la plage de longueurs d'onde appropriée pour l'excitation des marqueurs fluorescents de l'agent de lubrification ou de l'épilame à inspecter, et pour transmettre un flux lumineux d'excitation filtré ;
- un filtre dichroïque configuré pour réfléchir ledit flux lumineux d'excitation filtré et pour orienter ledit flux lumineux d'excitation filtré en direction d'un orifice d'inspection apte à être positionné en regard de ladite pièce d'horlogerie, ledit filtre dichroïque étant également configuré pour laisser passer un flux lumineux de réflexion émis par ladite pièce d'horlogerie sous excitation dudit flux lumineux d'excitation filtré ;
- un filtre d'émission configuré pour filtrer le flux lumineux de réflexion et transmettre un flux lumineux de fluorescence correspondant à la fluorescence des marqueurs fluorescents dudit agent de lubrification ou de l'épilame à inspecter ;
ledit boîtier optique comportant :
- une première interface de montage pour le montage de manière amovible de ladite source de lumière blanche portative sur le boîtier optique, ladite première interface de montage étant une interface de type femelle comportant une première cavité de forme cylindrique circulaire, de manière à correspondre à la forme circulaire de la source de lumière blanche portative formée par une lampe de poche ou une lampe stylo, la première interface de montage étant configurée de sorte que ledit flux lumineux d'excitation émis par ladite source de lumière blanche portative est dirigé en direction dudit filtre d'excitation ;
- une deuxième interface de montage pour le montage de manière amovible dudit dispositif grossissant portatif sur le boîtier optique, ladite deuxième interface de montage étant configurée de sorte que ledit dispositif grossissant se trouve en regard de l'orifice d'inspection.

Outre les caractéristiques évoquées dans le paragraphe précédent, l'ensemble modulaire portatif l'invention peut présenter une ou plusieurs caractéristiques complémentaires parmi les suivantes, considérées individuellement ou selon toutes les combinaisons techniquement possibles :
- la première interface de montage et/ou la deuxième interface de montage sont configurées pour réaliser un couplage mécanique et/ou par effet magnétique ; ainsi le montage est simple à réaliser ;
- ladite première interface de montage est configurée de sorte que le flux lumineux d'excitation émis par ladite source de lumière blanche portative est orienté perpendiculairement au filtre d'excitation ;
- ladite source de lumière blanche portative est une lampe de poche ou une lampe stylo ; ainsi, il n'est pas nécessaire de se procurer une source de lumière spécifique et onéreuse, une simple lampe de poche ou une lampe stylo permet d'exciter les marqueurs fluorescents d'un agent de lubrification fluorescent ;
- ledit dispositif grossissant portatif est une loupe d'horloger ;
- ledit filtre dichroïque présente une inclinaison de 45° par rapport au flux lumineux d'excitation filtré ;
- ledit boitier optique est réalisé en matériau polymère ou en matériau composite ; ainsi, les coûts de fabrication sont minimisés ;
- ledit boitier optique est réalisé en matériau polymère par impression 3D ; ainsi, la fabrication d'un tel boîtier optique et ensemble modulaire portatif est facilité, économique et accessible au plus grand nombre ; il suffit en effet d'intégrer ensuite des filtres optiques disponibles sur le marché ;
- la première interface de montage et/ou la deuxième interface de montage comportent des moyens de verrouillage pour verrouiller de manière réversible le couplage de ladite source de lumière blanche portative et/ou dudit dispositif grossissant sur ledit boîtier optique ; ainsi le montage des différents modules est sécurisé, ce qui permet de s'affranchir d'un désaccouplement non désiré lors de l'utilisation d'un tel ensemble modulaire portatif ;
- la deuxième interface de montage comporte des encoches pour faciliter le positionnement et/ou le démontage du filtre d'émission.

### Brève description des figures

Les buts, avantages et caractéristiques de la présente invention apparaîtront à la lecture de la description détaillée ci-dessous faisant référence aux figures suivantes :
- la figure 1 représente schématiquement une vue en perspective d'un exemple de réalisation d'un ensemble modulaire portatif selon l'invention ;
- la figure 2 représente schématiquement une vue éclatée de l'ensemble modulaire portatif illustré à la figure 1 ;
- la figure 3 représente schématiquement une vue en coupe sagittale de l'ensemble modulaire portatif illustré à la figure 1.

Dans toutes les figures, les éléments communs portent les mêmes numéros de référence sauf précision contraire.

### Description détaillée de l'invention

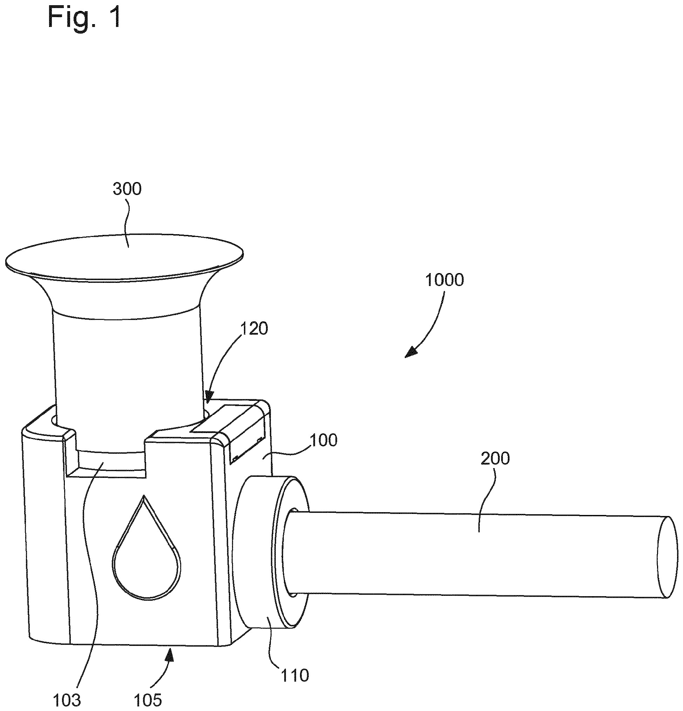

La figure 1 représente un exemple de réalisation d'un ensemble modulaire portatif 1000 selon l'invention, les différents modules de l'ensemble étant assemblés les uns aux autres sur cette figure.

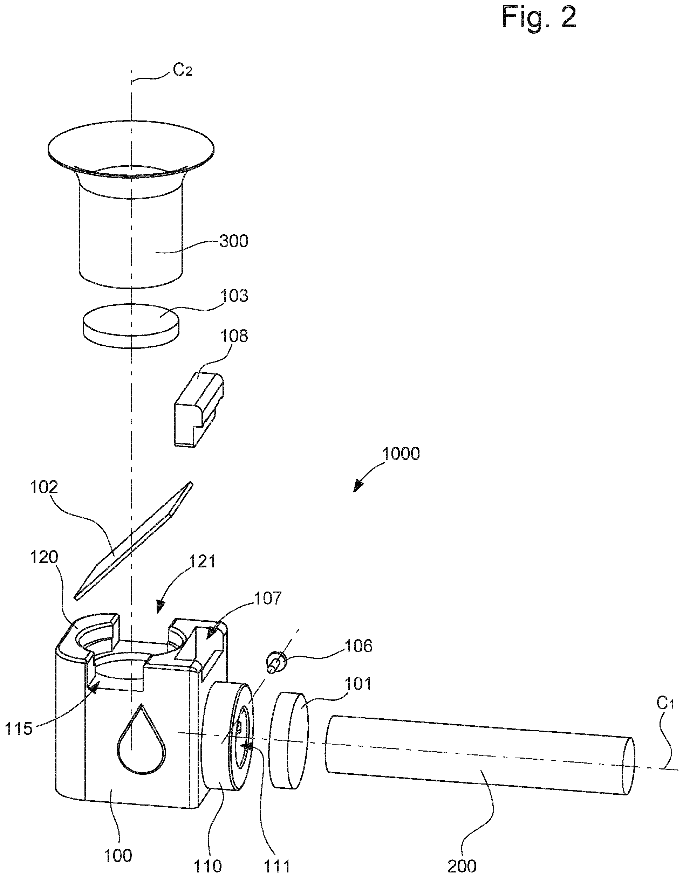

La figure 2 représente une vue éclatée de l'ensemble modulaire portatif 1000 selon l'invention.

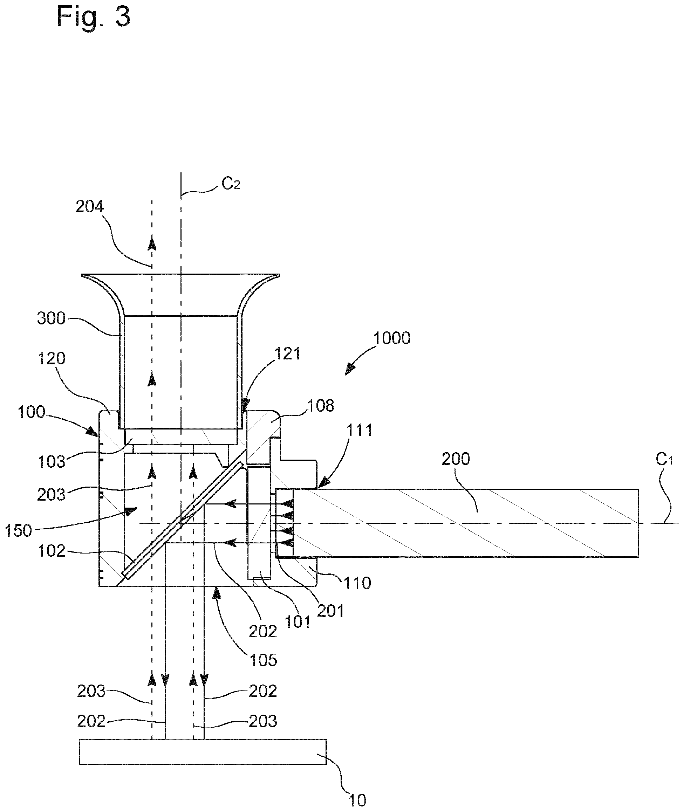

La figure 3 est une vue en coupe sagittale de l'ensemble modulaire portatif 1000 représenté à la figure 1.

En référence aux figures 1 à 3, l'ensemble modulaire portatif 1000 selon l'invention permet d'inspecter une pièce d'horlogerie 10 (schématisé sur la figure 3), comme par exemple un mouvement horloger.

L'ensemble modulaire portatif 1000 selon l'invention permet plus particulièrement d'inspecter la présence ou non, en quantité suffisante ou non, d'un agent de lubrification ou d'un épilame présentant des marqueurs fluorescents réagissant à une excitation lumineuse, au moyen d'une source de lumière blanche.

Dans la présente demande, on entend par agent de lubrification une graisse, une huile ou encore toute autre substance lubrifiante utilisée classiquement dans le domaine de l'horlogerie.

L'ensemble modulaire portatif 1000 est un assemblage de trois modules distincts.

Un premier module de l'ensemble modulaire portatif 1000 est formé par un boîtier optique 100 comportant les différents éléments optiques nécessaires pour l'excitation des marqueurs fluorescents et pour la transmission d'un flux lumineux de fluorescence résultant de cette excitation.

Un deuxième module de l'ensemble modulaire portatif 1000 est formé par une source de lumière blanche portative 200, d'usage courant, comme par exemple une lampe torche ou une lampe stylo. La source de lumière blanche portative 200 émet bien évidemment un flux lumineux de lumière blanche sur demande de l'utilisateur. Ce flux est appelé et référencé par la suite flux lumineux d'excitation 201.

Un troisième module de l'ensemble modulaire portatif 1000 est formé par un dispositif grossissant portatif 300 d'usage courant dans le domaine de l'horlogerie, comme par exemple une loupe d'horloger.

La combinaison de ces trois modules, et leur interfaçage de manière amovible et réversible permet de construire facilement et de manière économique un ensemble modulaire portatif 1000 pour l'inspection d'un agent de lubrification ou d'un épilame présentant des marqueurs fluorescents au moyen d'une source de lumière blanche. L'ensemble modulaire portatif 1000 selon l'invention permet de répondre aux différents inconvénients liés à l'utilisation de sources lumineuses nocives pour les yeux par l'utilisation d'une source de lumière blanche portative 200 d'usage courant facilement disponible dans un atelier d'horlogerie par exemple.

Le boîtier optique 100 comporte une première interface de montage 110 permettant l'accouplement, de manière amovible et réversible, de la source de lumière blanche portative 200.

La première interface de montage 110 est une interface de type femelle comportant une première cavité 111, par exemple de forme cylindrique, et plus particulièrement de forme cylindrique circulaire de manière à correspondre à la forme circulaire de la source de lumière blanche portative 200 formée par une lampe de poche ou une lampe stylo. La lampe de poche constitue la partie mâle de l'assemblage. La source de lumière blanche portative 200 est ainsi montée sur le boîtier 100 par introduction d'une portion d'extrémité de la source de lumière blanche portative 200 dans la première cavité 111.

La première cavité 111 de la première interface de montage 110 communique avec une chambre interne 150 ménagée à l'intérieur du boîtier optique 100.

Le boîtier optique 100 comporte une deuxième interface de montage 120 permettant l'accouplement, de manière amovible et réversible, du dispositif grossissant 300, de type loupe d'horloger.

La deuxième interface de montage 120 est également une interface de type femelle comportant une deuxième cavité 121, par exemple de forme cylindrique, et plus particulièrement de forme cylindrique circulaire, de manière à correspondre à la forme circulaire du dispositif grossissant 300 de type loupe d'horloger. Le dispositif grossissant 300 constitue la partie mâle de l'assemblage. Le dispositif grossissant 300 est ainsi monté sur le boîtier 100 par introduction d'une portion d'extrémité du dispositif grossissant 300 dans la deuxième cavité 121.

La deuxième cavité 121 de la deuxième interface de montage 120 communique avec également avec la chambre interne 150 du boîtier optique 100.

La chambre interne 150 est divisée en deux parties par un filtre dichroïque 102 positionné à l'intérieur de la chambre interne 150.

Le première cavité 111 s'étend selon un premier axe de révolution C1 et la deuxième cavité 121 s'étend selon un deuxième axe de révolution C2, la première cavité 111 et la deuxième cavité 121 étant ménagées de sorte que leur axe de révolution respectif C1, C2 soient orthogonaux et se croisent au niveau de la chambre interne 150.

Le filtre dichroïque 102 est positionné sensiblement au croisement de ces deux axes de révolution C1, C2, selon un angle de 45° par rapport à chacun de ces axes de révolution C1, C2.

Le boîtier optique 100 comporte un premier logement pour recevoir et porter un filtre d'excitation 101 présentant des propriétés pour sélectionner dans le flux lumineux d'excitation 201, provenant de la source de lumière blanche 200, une plage appropriée de longueurs d'onde pour l'excitation des marqueurs fluorescents de l'agent de lubrification ou de l'épilame à inspecter dans la pièce d'horlogerie 10, et pour transmettre un flux lumineux d'excitation filtré 202 dans la chambre interne 150.

Le filtre d'excitation 101 est avantageusement positionné perpendiculairement à l'axe de révolution C1 de la cavité 111 entre la chambre interne 150 et la première interface de montage 110 de sorte que le flux lumineux d'excitation filtré 202 puisse se propager à l'intérieur de la chambre interne 150 du boîtier optique 100 et arriver avec un angle proche de 45° sur le filtre dichroïque 102.

Le filtre d'excitation 101 forme ainsi l'interface entre la chambre interne 150 et la première cavité 111 de la première interface de montage 110 communiquant avec l'extérieur du boîtier optique 100.

Le filtre dichroïque 102 comporte des propriétés pour réfléchir le flux lumineux d'excitation filtré 202 arrivant dans la chambre interne 150 et pour l'orienter, de par son inclinaison, en direction d'un orifice d'inspection 105 situé en regard de la deuxième interface de montage 120 recevant le dispositif grossissant 300.

Le filtre dichroïque 102 présente également des propriétés pour laisser passer un flux lumineux de réflexion 203 résultant, émis par réflexion par la pièce d'horlogerie 10 sous l'excitation du flux lumineux d'excitation filtré 202.

Le boîtier optique 100 comporte un deuxième logement pour recevoir et porter un filtre d'émission 103 comportant des propriétés pour filtrer le flux lumineux de réflexion 203 et pour transmettre uniquement un flux lumineux de fluorescence 204 correspondant à la fluorescence des marqueurs fluorescents de l'agent de lubrification ou de l'épilame à inspecter.

Le filtre d'émission 103 est avantageusement positionné perpendiculairement à l'axe de révolution C2 de la cavité 121 entre la chambre interne 150 et la deuxième interface de montage 120 de manière à filtrer le flux lumineux de réflexion 203 et à transmettre un flux lumineux de fluorescence 204 à l'intérieur de la deuxième cavité 121 de la deuxième interface du montage 120 du boîtier 100 et au travers du dispositif grossissant 300.

Ainsi, le filtre d'émission 103 forme l'interface entre la chambre interne 150 et la deuxième cavité 121 de la deuxième interface de montage 120 communiquant avec l'extérieur du boîtier optique 100.

La première interface de montage 110 et la deuxième interface de montage 120 sont configurées pour réaliser un couplage mécanique.

Le couplage mécanique est par exemple assuré par la forme particulière et/ou le matériau de la première interface de montage 110 et de la deuxième interface de montage 120.

A titre d'exemple, la première interface de montage 110 et/ou la deuxième interface de montage 120 peuvent présenter au moins une portion en matériau polymère souples présentant des propriétés élastiques, ou encore en matériau élastomère permettant d'exercer un effort de retenue par réponse élastique ou viscoélastique du matériau autour la source de lumière blanche portative 200 et/ou du dispositif grossissant 300.

La première cavité 111 de la première interface de montage 110 et/ou la deuxième cavité 121 de la deuxième interface de montage 120 peuvent également présenter au niveau de la paroi périphérique délimitant la cavité 111, 121, un revêtement ou un joint élastomère pouvant améliorer la friction et/ou le maintien en position de la source de lumière blanche portative 200 ou du dispositif grossissant 300.

Selon une variante de réalisation ou en complément d'un couplage mécanique, tel que décrit précédemment, la première interface de montage 110 et/ou la deuxième interface de montage 120 sont configurées pour réaliser un couplage par effet magnétique. Dans ce cas, la première interface 110 et/ou la deuxième interface 120 comportent une portion en matériau magnétique ou encore un aimant permanent logé ou noyé au niveau de la première interface de montage 110 et/ou de la deuxième interface de montage 120. Cette variante de réalisation est particulièrement intéressante lorsque la source de lumière blanche portative 200 ou le dispositif grossissant 300 présente au moins une partie en matériau ferromagnétique. Ainsi, le couplage est assuré et facilité par l'attraction magnétique.

La première interface de montage 110 et la deuxième interface de montage 120 peuvent comporter en outre des moyens de verrouillage pour assurer provisoirement et de manière réversible le verrouillage des différents modules, respectivement de la source de lumière blanche portative 200 et du dispositif grossissant 300 en position sur le boîtier optique 100, et ainsi sécuriser le montage des modules.

Les moyens de verrouillage sont par exemple des moyens de verrouillage par vissage, des moyens de verrouillage à baïonnette.

Les moyens de verrouillage sont par exemple formés par un organe de verrouillage 106 présentant une tête de montage et un corps fileté coopérant avec un orifice taraudé ménagé selon une direction radiale au niveau d'une interface de montage.

Dans l'exemple de réalisation illustré aux figures 1 à 3, l'organe de verrouillage 106 coopère avec un orifice taraudé ménagé sur le pourtour de la première interface de montage 110 de manière à verrouiller, par vissage de l'organe de verrouillage 106, la source de lumière blanche portative 200 en position sur le boîtier optique 100.

Bien entendu, un tel organe de verrouillage en coopération avec un orifice taraudé peuvent être ménagés au niveau de la deuxième interface de montage 120 pour le verrouillage du dispositif grossissant 300 en position sur le boîtier optique 100

La première interface de montage 110 et la deuxième interface de montage 120 peuvent être identiques ou différents, et assurer un couplage identique ou différent. En outre, la première interface de montage 110 et la deuxième interface de montage 120 peuvent comporter des moyens de verrouillage identiques ou différents.

Bien que la présence de moyens de verrouillage supplémentaires permette de sécuriser le couplage et le maintien en position des différents modules 200, 300, notamment lors des différentes manipulations de l'ensemble modulaire portatif 1000, ces moyens de verrouillage ne sont pas obligatoires pour assurer le couplage et le maintien en position de la source de lumière blanche portative 200 ou du dispositif grossissant 300 sur le boîtier optique 1000. En effet, il suffit que la première interface de montage 110 et/ou la deuxième interface de montage 120 présente(nt) une cavité 111, 121 dont les dimensions sont configurées pour assurer l'introduction et le maintien en position de la source de lumière blanche portative 200 et du dispositif grossissant 300, avec les jeux de montage adéquats. Ainsi, avec une longueur suffisamment importante de la cavité 111, 121 et un diamètre adapté, la source de lumière blanche portative 200 et le dispositif grossissant 300 peuvent parfaitement être montés de manière amovible sur le boîtier optique 100 et maintenus en position durant l'utilisation de l'ensemble modulaire portatif 1000.

Dans l'exemple de réalisation illustré, le boîtier optique 100 comporte en outre une lumière 107 pour le positionnement du filtre d'excitation 101 dans son logement à l'intérieur du boîtier optique 100. La lumière 107 en forme de fente permet de faciliter le positionnement du filtre d'excitation 101 dans son logement entre la chambre interne 150 et la cavité 111 de la première interface de montage 110.

Le boîtier optique 100 peut comporter un bouchon 108 venant obturer la lumière 107 après le positionnement du filtre d'excitation 101. Le bouchon 108 peut être configuré pour exercer une légère compression sur le filtre d'excitation 101 de manière à assurer le maintien en position du filtre d'excitation dans son logement.

Le boitier optique 100 peut également comporter des encoches 115 ménagées au niveau des interfaces de montage 110, 120 pour faciliter le montage et/ou l'extraction des filtres optiques. Dans l'exemple de réalisation illustré aux figures 1 à 3, on a représenté de telles encoches 115 uniquement au niveau de la deuxième interface de montage 120 recevant le dispositif grossissant 300. Au niveau de la deuxième interface de montage 120, les encoches 115 permettent de faciliter la manipulation en vue du montage ou de l'extraction du filtre d'émission 103 positionné au fond de la deuxième cavité 121. Ces encoches 115 sont particulièrement utiles lorsque le filtre d'émission 103 à monter ou à extraire présente des dimensions inférieures aux dimensions de la deuxième cavité 121 recevant le dispositif grossissant 300.

Le boîtier optique 100 est avantageusement réalisé en matériau polymère ou en matériau composite. A titre d'exemple, le boîtier optique 100 est réalisé en matériau polymère, par exemple via un procédé d'impression en trois dimensions de manière à faciliter la fabrication et l'accessibilité d'un tel boîtier optique 100. Une fois le boîtier réalisé en impression 3D, il est facile d'intégrer les différents filtres optiques 101, 102, 103, disponibles sur le marché, dans différents logements prévus à cet effet au niveau du boîtier optique 100.

## Revendications

1. Ensemble modulaire portatif (1000) pour inspecter dans une pièce d'horlogerie (10) la présence d'un agent de lubrification ou d'un épilame présentant des marqueurs fluorescents, à partir d'un flux lumineux d'excitation de lumière blanche, ledit ensemble modulaire portatif (1000) comportant un boîtier optique (100), formant un premier module, une source de lumière blanche portative (200) émettant un flux lumineux d'excitation (201), formant un deuxième module, un dispositif grossissant portatif (300), formant un troisième module ;
ledit boîtier optique (100) portant :
- un filtre d'excitation (101) configuré pour sélectionner dans le flux lumineux d'excitation (201) de ladite source de lumière blanche portative (200), la plage de longueurs d'onde appropriée pour l'excitation des marqueurs fluorescents de l'agent de lubrification ou de l'épilame à inspecter, et pour transmettre un flux lumineux d'excitation filtré (202) ;
- un filtre dichroïque (102) configuré pour réfléchir ledit flux lumineux d'excitation filtré (202) et pour orienter ledit flux lumineux d'excitation filtré (202) en direction d'un orifice d'inspection (105) apte à être positionné en regard de ladite pièce d'horlogerie (10), ledit filtre dichroïque (102) étant également configuré pour laisser passer un flux lumineux de réflexion (203) émis par ladite pièce d'horlogerie (10) sous excitation dudit flux lumineux d'excitation filtré (202) ;
- un filtre d'émission (103) configuré pour filtrer le flux lumineux de réflexion (203) et transmettre un flux lumineux de fluorescence (204) correspondant à la fluorescence des marqueurs fluorescents dudit agent de lubrification ou de l'épilame à inspecter ; ledit boîtier optique (100) comportant :
- une première interface de montage (110) pour le montage de manière amovible de ladite source de lumière blanche portative (200) sur le boîtier optique (100), ladite première interface de montage (110) étant une interface de type femelle comportant une première cavité (111), de forme cylindrique circulaire, de manière à correspondre à la forme circulaire de la source de lumière blanche portative (200) formée par une lampe de poche ou une lampe stylo, la première interface de montage (110) étant configurée de sorte que ledit flux lumineux d'excitation (201) émis par ladite source de lumière blanche portative (200) est dirigé en direction dudit filtre d'excitation (101) ;
- une deuxième interface de montage (120) pour le montage de manière amovible dudit dispositif grossissant portatif (300) sur le boîtier optique (100), ladite deuxième interface de montage (120) étant configurée de sorte que ledit dispositif grossissant (300) se trouve en regard de l'orifice d'inspection (105).

2. Ensemble modulaire portatif (1000) pour inspecter dans une pièce d'horlogerie (10) la présence d'un agent de lubrification ou d'un épilame présentant des marqueurs fluorescents à partir d'un flux lumineux d'excitation de lumière blanche selon la revendication précédente, **caractérisé en ce que** la première interface de montage (110) et/ou la deuxième interface de montage (120) sont configurées pour réaliser un couplage mécanique et/ou par effet magnétique.

3. Ensemble modulaire portatif (1000) pour inspecter dans une pièce d'horlogerie (10) la présence d'un agent de lubrification ou d'un épilame présentant des marqueurs fluorescents à partir d'un flux lumineux d'excitation de lumière blanche selon l'une des revendications précédentes, **caractérisé en ce que** ladite première interface de montage (110) est configurée de sorte que le flux lumineux d'excitation (201) émis par ladite source de lumière blanche portative (200) est orienté perpendiculairement au filtre d'excitation (101).

4. Ensemble modulaire portatif (1000) pour inspecter dans une pièce d'horlogerie (10) la présence d'un agent de lubrification ou d'un épilame présentant des marqueurs fluorescents à partir d'un flux lumineux d'excitation de lumière blanche selon l'une des revendications précédentes, **caractérisé en ce que** ledit dispositif grossissant portatif (300) est une loupe d'horloger.

5. Ensemble modulaire portatif (1000) pour inspecter dans une pièce d'horlogerie (10) la présence d'un agent de lubrification ou d'un épilame présentant des marqueurs fluorescents à partir d'un flux lumineux d'excitation de lumière blanche selon l'une des revendications précédentes, **caractérisé en ce que** ledit filtre dichroïque (102) présente une inclinaison de 45° par rapport au flux lumineux d'excitation filtré (202).

6. Ensemble modulaire portatif (1000) pour inspecter dans une pièce d'horlogerie (10) la présence d'un agent de lubrification ou d'un épilame présentant des marqueurs fluorescents à partir d'un flux lumineux d'excitation de lumière blanche selon l'une des revendications précédentes, **caractérisé en ce que** ledit boitier optique (100) est réalisé en matériau polymère ou en matériau composite.

7. Ensemble modulaire portatif (1000) pour inspecter dans une pièce d'horlogerie (10) la présence d'un agent de lubrification ou d'un épilame présentant des marqueurs fluorescents à partir d'un flux lumineux d'excitation de lumière blanche selon l'une des revendications précédentes, **caractérisé en ce que** ledit boitier optique (100) est réalisé en matériau polymère par impression 3D

8. Ensemble modulaire portatif (1000) pour inspecter dans une pièce d'horlogerie (10) la présence d'un agent de lubrification ou d'un épilame présentant des marqueurs fluorescents à partir d'un flux lumineux d'excitation de lumière blanche selon l'une des revendications précédentes, **caractérisé en ce que** la première interface de montage (110) et/ou la deuxième interface de montage (120) comportent des moyens de verrouillage pour verrouiller de manière réversible le couplage de ladite source de lumière blanche portative (200) et/ou dudit dispositif grossissant (300) sur ledit boîtier optique (100).

9. Ensemble modulaire portatif (1000) pour inspecter dans une pièce d'horlogerie (10) la présence d'un agent de lubrification ou d'un épilame présentant des marqueurs fluorescents à partir d'un flux lumineux d'excitation de lumière blanche selon l'une des revendications précédentes, **caractérisé en ce que** la deuxième interface de montage (120) comporte des encoches (115) pour faciliter le positionnement et/ou le démontage du filtre d'émission (101).

## Patentansprüche

1. Tragbare modulare Einheit (1000) zum Untersuchen eines Uhrmachereistücks (10) auf das Vorhandensein eines Schmierstoffs oder eines Epilams, das fluoreszente Markierungen aufweist, aus einem Anregungslichtstrom aus weißem Licht, wobei die tragbare modulare Einheit (1000) ein optisches Gehäuse (100), das ein erstes Modul bildet, eine tragbare Weißlichtquelle (200), die einen Anregungslichtstrom (201) emittiert, die ein zweites Modul bildet, und eine tragbare Vergrößerungsvorrichtung (300), die ein drittes Modul bildet, beinhaltet;
wobei das optische Gehäuse (100) trägt:
- einen Anregungsfilter (101), der konfiguriert ist, um in dem Anregungslichtstrom (201) der tragbaren Weißlichtquelle (200) den Wellenlängenbereich auszuwählen, der zum Anregen der fluoreszenten Markierungen des zu untersuchenden Schmierstoffs oder des Epilams geeignet ist, und um einen gefilterten Anregungslichtstrom (202) zu übertragen;
- einen dichroitischen Filter (102), der konfiguriert ist, um den gefilterten Anregungslichtstrom (202) zu reflektieren, und um den gefilterten Anregungslichtstrom (202) in Richtung einer Untersuchungsöffnung (105) zu lenken, die imstande ist, gegenüber dem Uhrmachereistück (10) positioniert zu werden, wobei der dichroitische Filter (102) auch konfiguriert ist, um einen Reflexionslichtstrom (203) durchzulassen, der von dem Uhrmachereistück (10) unter Anregung des gefilterten Anregungslichtstroms (202) emittiert wird;
- einen Emissionsfilter (103), der konfiguriert ist, um den Reflexionslichtstrom (203) zu filtern und einen Fluoreszenzlichtstrom (204) zu übertragen, welcher der Fluoreszenz der fluoreszenten Markierungen des zu untersuchenden Schmierstoffs oder Epilams entspricht;
wobei das optische Gehäuse (100) beinhaltet:
- eine erste Montageschnittstelle (110) zum abnehmbaren Montieren der tragbaren Weißlichtquelle (200) an dem optischen Gehäuse (100), wobei die erste Montageschnittstelle (110) eine Schnittstelle vom Buchsentyp ist, der einen ersten Hohlraum (111) in kreiszylindrischer Form beinhaltet, um der Kreisform der tragbaren Weißlichtquelle (200) zu entsprechen, die durch eine Taschenlampe oder eine Stiftlampe gebildet wird, wobei die erste Montageschnittstelle (110) konfiguriert ist, sodass der von der tragbaren Weißlichtquelle (200) emittierte Anregungslichtstrom (201) in Richtung des Anregungsfilters (101) gerichtet ist;
- eine zweite Montageschnittstelle (120) zum abnehmbaren Montieren der tragbaren Vergrößerungsvorrichtung (300) an dem optischen Gehäuse (100), wobei die zweite Montageschnittstelle (120) konfiguriert ist, sodass sich die Vergrößerungsvorrichtung (300) der Untersuchungsöffnung (105) zugewandt befindet.

2. Tragbare modulare Einheit (1000) zum Untersuchen eines Uhrmachereistücks (10) auf das Vorhandensein eines Schmierstoffs oder eines Epilams, das fluoreszente Markierungen aufweist, aus einem Anregungslichtstrom aus weißem Licht nach dem vorstehenden Anspruch, **dadurch gekennzeichnet, dass** die erste Montageschnittstelle (110) und/oder die zweite Montageschnittstelle (120) konfiguriert sind, um eine mechanische Kopplung und/oder eine Kopplung durch magnetische Wirkung herzustellen.

3. Tragbare modulare Einheit (1000) zum Untersuchen eines Uhrmachereistücks (10) auf das Vorhandensein eines Schmierstoffs oder eines Epilams, das fluoreszente Markierungen aufweist, aus einem Anregungslichtstrom aus weißem Licht nach einem der vorstehenden Ansprüche, **dadurch gekennzeichnet, dass** die erste Montageschnittstelle (110) konfiguriert ist, sodass der von der tragbaren Weißlichtquelle (200) emittierte Anregungslichtstrom (201) senkrecht zum Anregungsfilter (101) ausgerichtet wird.

4. Tragbare modulare Einheit (1000) zum Untersuchen eines Uhrmachereistücks (10) auf das Vorhandensein eines Schmierstoffs oder eines Epilams, das fluoreszente Markierungen aufweist, aus einem Anregungslichtstrom aus weißem Licht nach einem der vorstehenden Ansprüche, **dadurch gekennzeichnet, dass** die tragbare Vergrößerungsvorrichtung (300) eine Uhrmacherlupe ist.

5. Tragbare modulare Einheit (1000) zum Untersuchen eines Uhrmachereistücks (10) auf das Vorhandensein eines Schmierstoffs oder eines Epilams, das fluoreszente Markierungen aufweist, aus einem Anregungslichtstrom aus weißem Licht nach einem der vorstehenden Ansprüche, **dadurch gekennzeichnet, dass** der dichroitische Filter (102) eine Neigung von 45° in Bezug auf den gefilterten Anregungslichtstrom (202) aufweist.

6. Tragbare modulare Einheit (1000) zum Untersuchen eines Uhrmachereistücks (10) auf das Vorhandensein eines Schmierstoffs oder eines Epilams, das fluoreszente Markierungen aufweist, aus einem Anregungslichtstrom aus weißem Licht nach einem der vorstehenden Ansprüche, **dadurch gekennzeichnet, dass** das optische Gehäuse (100) aus Polymermaterial oder Verbundmaterial hergestellt ist.

7. Tragbare modulare Einheit (1000) zum Untersuchen eines Uhrmachereistücks (10) auf das Vorhandensein eines Schmierstoffs oder eines Epilams, das fluoreszente Markierungen aufweist, aus einem Anregungslichtstrom aus weißem Licht nach einem der vorstehenden Ansprüche, **dadurch gekennzeichnet, dass** das optische Gehäuse (100) aus Polymermaterial durch 3D-Druck hergestellt ist.

8. Tragbare modulare Einheit (1000) zum Untersuchen eines Uhrmachereistücks (10) auf das Vorhandensein eines Schmierstoffs oder eines Epilams, das fluoreszente Markierungen aufweist, aus einem Anregungslichtstrom aus weißem Licht nach einem der vorstehenden Ansprüche, **dadurch gekennzeichnet, dass** die erste Montageschnittstelle (110) und/oder die zweite Montageschnittstelle (120) Verriegelungsmittel zum reversiblen Verriegeln der Kopplung der tragbaren Weißlichtquelle (200) und/oder der Vergrößerungsvorrichtung (300) am optischen Gehäuse (100) beinhalten.

9. Tragbare modulare Einheit (1000) zum Untersuchen eines Uhrmachereistücks (10) auf das Vorhandensein eines Schmierstoffs oder eines Epilams, das fluoreszente Markierungen aufweist, aus einem Anregungslichtstrom aus weißem Licht nach einem der vorstehenden Ansprüche, **dadurch gekennzeichnet, dass** die zweite Montageschnittstelle (120) Kerben (115) beinhaltet, um die Positionierung und/oder die Demontage des Emissionsfilters (101) zu erleichtern.

## Claims

1. Portable modular unit (1000) for inspecting in a timepiece (10) the presence of a lubricating agent, or of an epilame, having fluorescent markers, from an excitation luminous flux of white light, said portable modular unit (1000) comprising an optical housing (100), forming a first module, a portable white light source (200) emitting an excitation luminous flux (201), forming a second module, a portable magnifying device (300), forming a third module;
said optical housing (100) supporting:
- an excitation filter (101) configured to select in the excitation luminous flux (201) of said portable white light source (200), the wavelength range suitable for the excitation of the fluorescent markers of the lubricating agent or of the epilame to be inspected, and to transmit a filtered excitation luminous flux (202);
- a dichroic filter (102) configured to reflect said filtered excitation luminous flux (202) and to orient said filtered excitation luminous flux (202) in the direction of an inspection opening (105) capable of being positioned opposite said timepiece (10), said dichroic filter (102) also being configured to allow a reflection luminous flux (203) emitted by said timepiece (10) under excitation of said filtered excitation luminous flux (202) to pass through;
- an emission filter (103) configured to filter the reflection luminous flux (203) and transmit a fluorescence luminous flux (204) corresponding to the fluorescence of the fluorescent markers of said lubricating agent or of the epilame to be inspected;
said optical housing (100) comprising:
- a first mounting interface (110) for removably mounting said portable white light source (200) on the optical housing (100), the first mounting interface (110) being an interface of the female type comprising a first cavity (111), of circular cylindrical shape, in such a way as to correspond to the circular shape of the portable white light source (200) formed by a torch or a penlight, the first mounting interface (110) being configured so that said excitation luminous flux (201) emitted by said portable white light source (200) is directed in the direction of said excitation filter (101);
- a second mounting interface (120) for removably mounting said portable magnifying device (300) on the optical housing (100), said second mounting interface (120) being configured so that said magnifying device (300) is opposite the inspection opening (105).

2. Portable modular unit (1000) for inspecting in a timepiece (10) the presence of a lubricating agent or of an epilame having fluorescent markers from an excitation luminous flux of white light according to the preceding claim, **characterised in that** the first mounting interface (110) and/or the second mounting interface (120) are configured to perform a mechanical and/or magnetic effect coupling.

3. Portable modular unit (1000) for inspecting in a timepiece (10) the presence of a lubricating agent or of an epilame having fluorescent markers from an excitation luminous flux of white light according to one of the preceding claims, **characterised in that** said first mounting interface (110) is configured so that the excitation luminous flux (201) emitted by said portable white light source (200) is oriented perpendicularly to the excitation filter (101).

4. Portable modular unit (1000) for inspecting in a timepiece (10) the presence of a lubricating agent or of an epilame having fluorescent markers from an excitation luminous flux of white light according to one of the preceding claims, **characterised in that** said portable magnifying device (300) is a watchmakers' eyeglass.

5. Portable modular unit (1000) for inspecting in a timepiece (10) the presence of a lubricating agent or of an epilame having fluorescent markers from an excitation luminous flux of white light according to one of the preceding claims, **characterised in that** said dichroic filter (102) has an inclination of 45° in relation to the filtered excitation luminous flux (202).

6. Portable modular unit (1000) for inspecting in a timepiece (10) the presence of a lubricating agent or of an epilame having fluorescent markers from an excitation luminous flux of white light according to one of the preceding claims, **characterised in that** said optical housing (100) is made of polymer material or of composite material.

7. Portable modular unit (1000) for inspecting in a timepiece (10) the presence of a lubricating agent or of an epilame having fluorescent markers from an excitation luminous flux of white light according to one of the preceding claims, **characterised in that** said optical housing (100) is made of polymer material by 3D printing

8. Portable modular unit (1000) for inspecting in a timepiece (10) the presence of a lubricating agent or of an epilame having fluorescent markers from an excitation luminous flux of white light according to one of the preceding claims, **characterised in that** the first mounting interface (110) and/or the second mounting interface (120) comprise locking means to reversibly lock the coupling of said portable white light source (200) and/or of said magnifying device (300) on said optical housing (100).

9. Portable modular unit (1000) for inspecting in a timepiece (10) the presence of a lubricating agent or of an epilame having fluorescent markers from an excitation luminous flux of white light according to one of the preceding claims, **characterised in that** the second mounting interface (120) comprises notches (115) to facilitate the positioning and/or the dismantling of the emission filter (101).
